# EUROPÄISCHE PATENTSCHRIFT

(11) **EP 2 954 864 B1**
(45) Veröffentlichungstag und Bekanntmachung des Hinweises auf die Patenterteilung: **21.08.2019**
(21) Anmeldenummer: 15163021.7
(22) Anmeldetag: 11.06.2014
(51) Int. Cl.: A61B 17/80, A61B 17/56, A61F 2/28, A61F 2/30, A61B 90/00

(54) **VERFAHREN ZUM FERTIGEN EINES PATIENTENSPEZIFISCHEN AUGENHÖHLENABDECKGITTERS**
METHOD FOR PRODUCING A PATIENT-SPECIFIC EYE CAVITY COVER GRID
PROCÉDÉ DE FABRICATION D'UNE GRILLE DE RECOUVREMENT DES ORBITES SPÉCIFIQUE AU PATIENT

(43) Veröffentlichungstag der Anmeldung: 16.12.2015
(62) Teilanmeldung aus: 14172026.8
(73) Patentinhaber: Karl Leibinger Medizintechnik GmbH & Co. KG, 78570 Mühlheim/Donau (DE)
(72) Erfinder: Reinauer, Frank, 78570 Muehlheim (DE); Scheunemann, Oliver, 78570 Muehlheim (DE)
(74) Vertreter: Winter, Brandl, Fürniss, Hübner, Röss, Kaiser, Polte - Partnerschaft mbB

(56) Entgegenhaltungen:
- US-A1- 2003 109 784
- US-A1- 2011 319 745
- US-A1- 2012 010 711

## Beschreibung

Die Erfindung betrifft ein Verfahren zum Herstellen eines patientenspezifisch angepassten Augenhöhlenabdeckgitters. Erfindungsgemäß werden dabei einzelne Schritte durchlaufen, die zeitlich nacheinander ablaufen. So wird in einem Schritt ein (3D-)Primärmodell der abzudeckenden oder ersetzenden Knochenstruktur im Bereich einer Augenhöhle eines zu behandelnden (menschlichen oder tierischen) Patienten geschaffen. Ein weiterer Schritt betrifft das Festlegen eines für die maximale räumliche Ausdehnung des geplanten Augenhöhlenabdeckgitters repräsentativen Grenzbereichs, zumindest in puncto seiner flächigen Ausdehnung. Ein weiterer Schritt betrifft das Überführen eines (2D-)Sekundärmodells so auf das (3D-)Primärmodell, etwa innerhalb eines vordefinierten / beliebigen Grenzbereichs, derart, dass die geometrische Beschaffenheit des Primärmodells auf die Ausgangsform des ursprünglichen Sekundärmodells übertragen wird und zu einem (3D-)Tertiärmodell führt. Nach diesen Schritten wird auf Basis der Daten nach einem Separierschritt vom ursprünglichen (3D-)Primärmodell, also auf Basis des (3D-)Tertiärmodells, die Fertigung des Augenhöhlenabdeckgitters durchgeführt. Im Kern steht also, dass ein 2D-Template virtuell auf eine Unterlage projiziert wird, wobei die Unterlage patientenspezifisch nachgebildete Erhabenheiten aufweisen kann bzw. den patientenspezifischen Besonderheiten nachgebildet ist.

Die Erfindung betrifft somit ein Verfahren zum Herstellen eines patientenspezifisch angepassten Augenhöhlenabdeckgitters für alle vier Augenhöhlenwände. Eine Ankoppelungsfähigkeit an beliebige Defekte im Mittelgesicht ist gegeben.

Die Offenbarung betrifft auch ein patientenspezifisches Augenhöhlenabdeckgitter zu allen vier Augenhöhlenwänden, insbesondere nach Art eines "dreidimensionalen Orbita Meshes", mit einem geschwungenen / S-förmig gebogenen / mehrfach gekrümmten Hauptkörper, der eine äußere, normalerweise umlaufend geschlossene Abschlusskante / Einfassung aufweist, wobei der Hauptkörper eine Unterseite aufweist, die im implantierten Zustand dem oder den die Augenhöhle ausformenden Knochen zugewandt ist und der Hauptkörper eine der Unterseite abgewandte Oberseite aufweist.

Unter einem Gitter wird eine Anordnung aus länglichen Teilen in regelmäßigen oder unregelmäßigen Abständen subsumiert. Es kann eine netzartig ausgestaltete Flächenstruktur haben.

Aus dem Stand der Technik sind bereits Augenhöhlenabdeckgitter bekannt, wie beispielsweise aus der EP 1 965 735 B1. Dort wird ein Implantat zur Verwendung als Ersatz eines Orbitabodens eingesetzt. Das Implantat ist als Augenhöhlenabdeckgitter ausgestaltet, liegt also am Orbitaboden auf. So ein Implantat, wie ein "Mesh" bzw. Gitter, kann auch zur seitlichen Orbitawandrekonstruktion verwendet werden. Es kann auch freitragend eingesetzt werden und muss nicht unbedingt am Boden aufliegen. In der besagten Druckschrift wird ein Implantat für die Verwendung als Ersatz eines Augenhöhlengrunds und optional auch einer medialen und lateralen Augenhöhlenwandung in der Form einer einstückig vorgeformten Platte, die einen ersten Abschnitt, einen zweiten Abschnitt und einen dritten Abschnitt umfasst, vorgestellt, wobei der erste Abschnitt gemäß einem Augenhöhlengrund und der zweite Abschnitt gemäß einer medialen Seitenwandung geformt sind und der erste Abschnitt und der zweite Abschnitt an einer ersten vorbestimmten Linie anliegen, wobei der dritte Abschnitt zur Befestigung des Implantats am vorderen Augenhöhlenrand angeordnet ist, wobei als besonders herausgestellt ist, dass die erste vorbestimmte Linie in der besagten Druckschrift als Bruchlinie definiert ist, entlang welcher ein Arzt ein Segment leicht entfernen kann.

Gitterartig ausgebildete Platten sind auch in ähnlicher Form zum Einsatz an anderen Teilen des Körpers bekannt.

So offenbart beispielsweise die DE 197 46 396 A1 ein Gitter für die Fixierung von Knochenteilen oder für die Überbrückung von Knochenfehlstellen. Ein solches Gitter kann auch am Schädel eingesetzt werden. Letztlich wird in dieser deutschen Druckschrift ein Gitter zur Anwendung im Schädel- und Kieferbereich vorgeschlagen, bestehend aus biokompatiblen Materialien mit einer netzartigen Struktur und mit Ausnehmungen zur Aufnahme von Knochenschrauben, mit denen das Gitter am Knochen befestigbar ist. Die Stege bilden mäanderförmige, durchgehende, periodische Stegreihen entlang der Hauptachse des Gitters.

Das Augenhöhlenabdeckgitter, also jene Vorrichtung, die zum In-Kontakt-Gelangen mit dem Orbitaboden vorgesehen ist, darf bei der Anbringung am Knochen die Augapfelaufnahme nicht behindern. Jene Augapfelaufnahme ist jedoch nicht sphärisch, sondern erstreckt sich länglich, insbesondere S-förmig.

Weiterhin offenbart die US 2012/010711 A1 ein Verfahren zum Bilden eines patientenspezifischen Implantats.

Die US 2003/109784 A1 offenbart ein Verfahren zum Herstellen von Formblechen als Prothese.

Abschließend offenbart die US 2011/319745 eine patientenangepasste chirurgische Führung und eine Methode, um diese zu benutzen.

Den aus dem Stand der Technik bekannten Verfahren liegt eine Vielzahl an Verfahrensschritten zugrunde. Außerdem ist häufig eine Nachbearbeitung des Implantats notwendig.

Es ist die Aufgabe der Erfindung, hier Abhilfe zu bieten und ein Verfahren vorzustellen, das ein einfaches Herstellen eines Augenhöhlenabdeckgitters ermöglicht. Letztlich soll auch ein Verfahren vorgestellt werden, um schnell und präzise Verletzungen des Orbitabodens und der seitlichen Orbitawände dauerhaft zu behandeln, mit der Option der Ankopplung an z.B. ebenfalls zu ersetzende Mittelgesichtsstrukturen - wie z.B. im Falle ausgedehnter tumorbedingter Resektionsdefekte. Ferner soll eine möglichst optimale und patientenspezifische Ausgangsstruktur eines Augenhöhlenabdeckgitters dem Operateur zur Verfügung gestellt werden, insbesondere ein solches Augenhöhlenabdeckgitter, was nicht zu groß ist, bereits vorangepasst an den jeweiligen zu behandelnden Defekt ist und einfach feinanpassbar ist.

Diese Aufgabe wird erfindungsgemäß mit einem Verfahren gelöst, das sich zum Herstellen eines patientenspezifisch angepassten Augenhöhlenabdeckgitters eignet, wobei ein 3D-Primärmodell der abzudeckenden oder ersetzenden Knochenstruktur im Bereich einer Augenhöhle eines zu behandelnden Patienten geschaffen wird, danach ein für die maximale räumliche Ausdehnung des geplanten Augenhöhlenabdeckgitters repräsentativer Grenzbereich festgelegt wird, danach ein 2D-Sekundärmodell innerhalb des Grenzbereichs so auf das 3D-Primärmodell überführt wird, dass die geometrische Beschaffenheit des 3D-Primärmodells auf die Ausgangsform des ursprünglichen 2D-Sekundärmodells übertragen zu einem 3D-Tertiärmodell führt, auf dessen Basis die Fertigung des Augenhöhlenabdeckgitters basiert.

Weiterhin gilt, dass bei einem gattungsgemäßen Augenhöhlenabdeckgitter auf der Oberseite wenigstens ein optisch erkennbarer Kanal zum Darstellen wenigstens eines Insertionsvektors ausgeprägt sein kann.

Auf diese Weise kann das Augenhöhlenabdeckgitter einfacher und präziser an bzw. in den Patienten verbracht werden. Der Patient kann ein Säugetier sein, insbesondere ein Mensch oder ein (Säuge-)Tier. Dabei ist das Augenhöhlenabdeckgitter zwischen einem die Augenhöhle ausfüllenden Weichgewebe und die die eigentliche Augenhöhle bildende Knochenstruktur zu verbringen. Das Augenhöhlenabdeckgitter, ist dann ein Implantat, das auf der Knochenstruktur aufliegt, zumindest mit möglichst drei Punkten in Kontakt dort befindlich ist und von Weichgewebe nach der Implantierung bedeckt ist. Natürlich ist es möglich auch weniger als drei Auflagepunkte zu nutzen. Ist das Augenhöhlenabdeckgitter erfindungsgemäß ausgestaltet, kann das Einsetzen präziser, atraumatisch und verletzungsfrei / verletzungsfreier erfolgen; insbesondere besteht Objektivierbarkeit für die Lagekontrolle in röntgenbasierten Bildgebungsverfahren. Die Verträglichkeit des Augenhöhlenabdeckgitters beim Patienten wird wesentlich verbessert. Der Tragekomfort wird erhöht.

Vorteilhafte Ausführungsformen sind in den Unteransprüchen beansprucht und werden nachfolgend näher erläutert.

Zweckmäßig ist es, wenn bei der Fertigung generative Verfahren, wie Sinterverfahren, und/oder CNC -, Fräs- und / oder Spritzgussverfahren angewandt werden. Besonders bewährt haben sich Laser-Sinterverfahren, wie SLM-Verfahren, also Selective Laser Melting-Verfahren.

Dabei ist es von Vorteil, wenn das Augenhöhlengitter nur aus einem oder mehreren Metallwerkstoff(en) oder nur aus Kunststoff oder einem Gemisch aus Metall und Kunststoff besteht. Es können zusätzlich Keramikbestandteile hinzugefügt sein. Auch kann das Augenhöhlengitter komplett aus Keramik hergestellt sein. Dabei bietet sich Zirkoniumoxid oder Hydroxylapatit.

Es ist auch zweckmäßig, wenn das Sekundärmodell aus mehreren Schichten aufgebaut / zusammengesetzt wird / ist.

Ein vorteilhaftes Ausführungsbeispiel ist auch dadurch gekennzeichnet, dass beim Überführen oder Planen / Auslegen des Primär- oder Sekundärmodells eine gewollte Abweichung von den 3D-Patientendaten akzeptiert wird / eingesetzt ist, um den Rand des Augenhöhlengitters operateurspezifisch und / oder für den Implantiervorgang optimiert zu gestalten.

Während ein Spitzenbereich des Augenhöhlengitters zum Kontaktieren des Knochens vorbereitet wird, etwa gekrümmt wird, bspw. stärker gekrümmt wird als durch die 3D-Patientendaten vorgegeben, und / oder der vordere Rand des Augenhöhlengitters als Griffbereich für den (manuellen) Operateurzugriff vorbereitet wird, etwa so gekrümmt wird, bspw. stärker gekrümmt als durch die 3D-Patientendaten vorgegeben, so kann ein besonders sicher handhabbares Augenhöhlengitter geschaffen werden.

Eine besonders gute Abstimmung lässt sich erreichen, wenn eine an der Abschlusskante vorhandene Kordel eine Dicke von ca. 0,3 bis ca. 0,7 mm, z.B. ca. 0,5 mm aufweist und die innerhalb davon vorhandene Fläche des Hauptkörpers eine Dicke von ca. 0,1 mm bis ca. 0,5 mm, etwa 0,3 mm aufweist. Die Werte sind Zirkawerte und können mit einer Abweichung von 10 % oder 20 % behaftet sein.

Dies gilt auch für eine Kordel, die zwischen ca. 0,1 bis ca. 0,3 mm, etwa 0,2 mm dicker als die dazu belastete Fläche des Hauptkörpers ausgebildet ist.

Es ist von Vorteil, wenn eine Binnenmatrix bzgl. einer oder mehrerer Faktoren aus Struktur, Geometrie, Porengröße und biomechanischen Eigenschaften gezielt / frei gewählt ist, etwa in Anpassung / Nachahmung / Verbesserung des zu ersetzenden / ergänzenden Materials der jeweiligen anatomischen Region des Patienten.

Auch ist es von Vorteil, wenn eine patientenspezifische Identifizierung, etwa nach Art eines Barcodes und / oder einer Zeichenfolge aus Buchstaben und / oder Ziffern, auf das Augenhöhlengitter aufgebracht wird, etwa während des Fertigungsschritts aus jenem Material, das das Augenhöhlengitter bildet, vorzugsweise in einem (Laser-) Sinterverfahren als Erhabenheit, insbesondere zur Wiedergabe des Patientennamens und / oder der Implantierungsposition /-lage.

So ist es von besonderem Vorteil, wenn der Kanal zwei Punkte linear verbindet, also wenigstens abschnittsweise linear ausgestaltet ist, oder besser in Gänze linear ausgeformt ist.

So ist es weiterhin von Vorteil, wenn der Hauptkörper als ein stegausbildendes, perforiertes Bauteil ausgebildet ist. Die Adaptierbarkeit an den bspw. menschlichen Körper wird dadurch verbessert. Außerdem ist die Gefahr der Ausbildung eines abgeschlossenen Raumes reduziert, d.h. im Falle von z.B. Nachblutungen erlauben die Gitteröffnungen das Ablaufen von Blut in benachbarten Nasennebenhöhlen.

Wenn im Hauptkörper Stege so angeordnet sind, dass sich durch den Hauptkörper längliche, sich in der durch den Hauptkörper aufgespannten Fläche verlaufende Durchgangsschlitze ziehen, insbesondere solche, die sich von der Unterseite zur Oberseite des Hauptkörpers erstrecken, so wird die Verträglichkeit des Augenhöhlenabdeckgitters beim Patienten verbessert, Gewicht verringert, Material eingespart, Kosten reduziert und die Ausbildung eines Abflusssystems kreierbar.

Es ist auch zweckmäßig, wenn die Schlitze (nahezu) orthogonal zur Abschlusskante / Einfassung ausgerichtet sind und / oder zueinander äquidistant verteilt sind. Der Einsetzvorgang wird dadurch besser kontrollierbar. Auch andere Vorteile, wie das Erhalten eines besonders rigiden Implantats, können sich einstellen.

Wenn der Kanal zwei sich von der Oberseite erhaben abhebende und zueinander gleich beabstandet verlaufende Kanalwände besitzt, so kann ein Kontrollinstrument einfach auf den Kanal aufgesetzt werden und beim Einsetzen kontrollierend fungieren. Durch die erhaben ausgestalteten Kanalwände wird ein Verlassen des Kontrollinstrumentes nach außerhalb des Kanals wirkungsvoll verhindert.

Eine Strukturschwächung wird vermieden, wenn der Kanal zwischen den Kanalwänden einen Kanalgrund aufweist, der durch die Oberseite des Hauptkörpers gebildet ist oder wenigstens in der durch die Oberfläche gebildeten Fläche verläuft. Auch kann die Fertigung dann kostengünstig durchgeführt werden.

Es ist auch von Vorteil, wenn der vorzugsweise unterbrochen / durchgehend oder abschnittsweise unterbrochen ausgestaltete Kanal von einem vorderen Rand, der einem Operateur nächstgelegen ist, bis zu einem Spitzenbereich verläuft, der im implantierten Zustand einem Sehnerv / Sehkanal nächstgelegen oder nahegelegen ist. Das Aufsetzen des Spitzenbereichs am Knochen wird unter Vermeidung einer Irritation oder Beschädigung des Sehnervs / des Sehkanals vereinfacht. Auch wird es einfacher, den Spitzenbereich in Kontakt mit einem tief im Inneren von Weichgewebe vorhandenem Knochenabschnitt zu bringen. Dabei ist es von Vorteil, wenn das Implantat im Spitzenbereich zusätzlich noch überbogen wird, um einen größeren Abstand zum Sehnerv zu erhalten.

Der Einsetzvorgang wird noch präziser durchführbar, wenn ein zweiter Kanal zum Darstellen eines weiteren Insertionsvektors vorhanden ist. Der zweite Kanal kann dann ähnlich oder identisch zum ersten Kanal ausgebildet sein und gibt den Übergang zwischen dem Orbitaboden und der seitlichen Wand an.

Insbesondere kann der erste Kanal zum zweiten Kanal quer verlaufend ausgerichtet sein, insbesondere um einen Winkel a, der im Bereich von 20° bis 40°, insbesondere 22,5°, liegt, winkelig versetzt sein.

Wenn die Kanalränder eine Führung für ein zwischen ihnen eingesetztes und entlang geschobenes Kontrollinstrument bilden, wird ein Verrutschen des Kontrollinstrumentes effizient vermieden.

Damit das Einsetzen des Augenhöhlenabdeckgitters / des Implantats abschnittsweise unterbrochen und / oder kontrolliert werden kann, ist es von Vorteil, wenn zwischen den Kanalwänden und / oder auf / in dem Kanalgrund ein von dem Kontrollinstrument vorzugsweise haptisch oder taktil erfassbarer Navigations-Stopp in Form einer Erhebung oder Vertiefung vorhanden / ausgebildet ist, und vorzugsweise mehrere Navigations-Stopps pro Kanal ausgebildet sind, wobei in den Kanälen gleich viele oder unterschiedlich viele Navigations-Stopps pro Kanal vorhanden sind, etwa im zweiten Kanal ein Navigations-Stopp weniger als im ersten Kanal. Navigations-Stopps können auf dem kompletten Körper des Implantats gesetzt werden, vorzugsweise jedoch auf dem Kanal. Die Navigations-Stopps sind dabei als intraoperativ anzusteuernde Landmarken definiert. Ferner ist eine Trajektorienplanung realisierbar, die die eingelassenen Insertionsvektoren aufnimmt und verabfolgen lässt.

Es ist auch von Vorteil, wenn der erste Kanal parallel zu einer Sagittalebene des zu behandelnden Patienten ausgerichtet ist und / oder der zweite oder erste Kanal parallel zu einer Schrägsagittalebene des zu behandelnden Patienten ausgerichtet ist. Eine im dreidimensionalen Raum geschwungene Einsetzbewegung lässt sich dann einfacher vom Operateur auf ihre Präzision kontrollieren.

Wenn der Spitzenbereich eine andere Krümmung als der überwiegende Teil des Hauptkörpers, insbesondere als der direkt anschließende / benachbarte Bereich des Hauptkörpers, aufweist, vorzugsweise konvex gewölbt ist, d.h. beispielsweise in Richtung des Knochens zunehmend gekrümmt / verlaufend ist, so wird eine verletzungsfreie Handhabung des Augenhöhlengitters beim Implantieren in den bspw. menschlichen Körper erleichtert.

Es ist zweckmäßig, wenn sich der erste Kanal und der zweite Kanal im Spitzenbereich treffen oder nahezu treffen. Natürlich ist es möglich, dass der Schnittpunkt der beiden Kanäle außerhalb des Implantats liegt, bspw. um ca. 1 mm bis ca. 4 mm, insbesondere ca. 1,3 mm außerhalb der Abschlusskante des Augenhöhlengitters vorhanden ist.

Ein vorteilhaftes Ausführungsbeispiel ist auch dadurch gekennzeichnet, dass eine für die auf dem Hauptkörper vorhandenen Abmessungen repräsentative Längenskala aufgebracht ist.

Dabei ist eine Weiterbildung dadurch gekennzeichnet, dass die für die Längenskala relevanten Zeichen, wie Ziffern, neben einem der Kanäle, etwa links oder rechts neben dem ersten oder zweiten Kanal, auf / in der Oberseite eingebracht / aufgebracht sind, vorzugsweise nach Art eines (kalibrierten) Lineals. Insbesondere lässt sich die Distanz vom Spitzenbereich dadurch markieren. Die Abstände von ca. 15 mm, ca. 25 mm und ca. 35 mm sowie Zwischenwerte wie ca. 10 mm, ca. 20 mm und / oder ca. 30 mm lassen sich dann einfach kennzeichnen. Die Markierungen können in 5 mm Abständen gesetzt werden. Damit die Anpassung an den Patienten verbessert wird, ist es von Vorteil, wenn der vordere Rand auf der Oberseite eine konvexe Wölbung und / oder auf der Unterseite eine konkave Wölbung aufweist. Auch lässt sich dann das Greifen für einen Operateur erleichtern. Insbesondere das manuelle Halten des Augenhöhlengitters am vorderen Rand mit den Fingern des Operateurs wird erleichtert.

Die Befestigung des Augenhöhlengitters am Knochen wird präziser, wenn im vorderen Rand wenigstens ein Durchgangsloch zum Aufnehmen von einer das Augenhöhlengitter am Knochen befestigenden Schraube vorhanden ist, vorzugsweise mehrere Durchgangslöcher für mehrere Schrauben und / oder das Durchgangsloch quer zur Ober- und / oder Unterseite des Hauptkörpers (im Bereich des Durchgangsloches) ausgerichtet ist, um einem Bohrvektor zu folgen. Auch wird ein Verrutschen des Augenhöhlengitters relativ zum Knochen dadurch wirkungsvoll verhindert. Dabei hat es sich bewährt, im Durchgangsloch einen Schraubenvektor zu berechnen, um vorher zu wissen, wo das meiste Knochenangebot vorhanden ist und dieses sinnvoll zu nutzen.

Es ist von Vorteil, wenn ein Tränenwegsbereich physisch vordefiniert / ausgebildet ist.

Auch ist es vorteilhaft, wenn der Hauptkörper als Platte, Netz und / oder Mehrschichtbauteil ausgebildet ist.

Wenn die Durchgangsschlitze oder Perforationen als Abschlusssystem ausgelegt sind, wird die Verträglichkeit des Augenhöhlengitters beim Patienten verbessert, insbesondere, um eine Abflussmöglichkeit bei eine möglichen Nachblutung zu schaffen.

Ein vorteilhaftes Ausführungsbeispiel ist auch dadurch gekennzeichnet, dass die Abschlusskante aus dickerem Material als der (überwiegende) Rest des Hauptkörpers nach Art einer atraumatisch wirkenden Kordel ausgeformt ist.

Als vorteilhaft für die Verträglichkeit hat es sich auch herausgestellt, wenn das Augenhöhlengitter auf einen spezifischen Patienten vorbereitet und / oder angepasst ist.

Wenn die Durchgangsschlitze so angeordnet sind, dass ein unbeabsichtigtes Umklappen von Teilbereichen des Hauptkörpers erschwert oder ausgeschlossen ist, wird ein besonders belastbares / rigides Implantat schaffbar / geschaffen. Insbesondere ist es von Vorteil, wenn eine mediale Wand nur so hoch ist, wie patientenspezifisch notwendig, aber möglichst hoch ausgestaltet ist, wenn die Notwendigkeit dafür besteht.

Die Patientenverträglichkeit wird verbessert, wenn der Spitzenbereich umgedreht schneeschieberartig ausgebildet ist, um eine vom Sehnerv wegragende Krümmung zu erhalten.

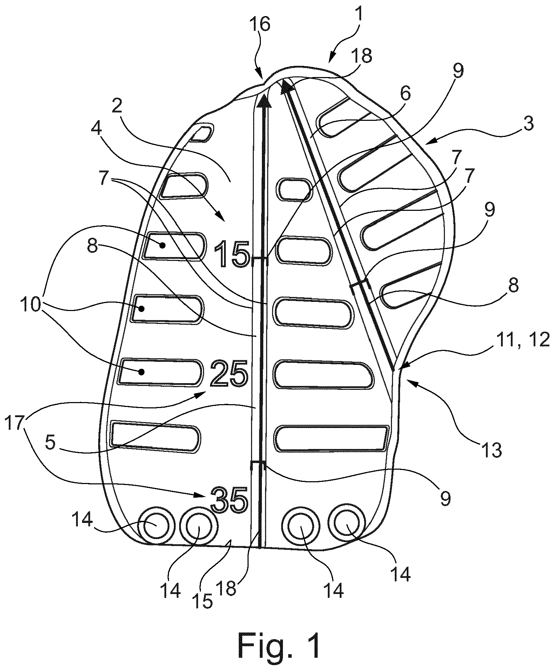

Die Erfindung wird nachfolgend auch mit einer Zeichnung näher erläutert, in der in der einzigen Figur, nämlich der Fig. 1, eine Draufsicht auf ein erfindungsgemäßes Augenhöhlenabdeckgitter wiedergegeben ist. Die Zeichnung ist lediglich schematischer Natur und dient nur dem Verständnis der Erfindung.

Das erfindungsgemäße Verfahren betrifft auch die Fertigung eines solchen Augenhöhlenabdeckgitters.

Es ist auch möglich mehrere Augenhöhlenabdeckgitter übereinander zu verbauen / implantieren. Die einzelnen kombinierten und sich wenigstens teilweise überdeckenden Augenhöhlenabdeckgitter können für sich genommen auch unterschiedliche Formen aufweisen. Beispielsweise kann eine zylinder- oder dreiecksförmige Form favorisiert sein.

In Fig. 1 ist eine erste Ausführungsform eines Augenhöhlenabdeckgitters 1, wie es mittels des erfindungsgemäßen Verfahrens herstellbar ist, dargestellt. Das Augenhöhlenabdeckgitter 1 ist als "Orbita Mesh" ausgebildet. Es weist einen mehrfach geschwungenen / gebogenen / gekrümmten Hauptkörper 2 auf.

Der Hauptkörper 2 weist auf seiner Außenseite eine im Wesentlichen umlaufende / geschlossene äußere Abschlusskante / Einfassung 3 auf. Eine Unterseite des Augenhöhlenabdeckgitters 1, insbesondere des Hauptkörpers 2 und der Abschlusskante / Einfassung 3 ist patientenspezifisch gestaltet. Somit ist die Innen- und Außenstruktur patientenspezifisch. Die Binnenmatrix des Hauptkörpers und das verwendete Material, bspw. einer Titanlegierung, ist in puncto Biegesteifigkeit und / oder Elastizitätsmodul an die angrenzende patientenspezifische anatomische Region angepasst, möglichst gleichnachbildend ausgesucht.

Auf diese Weise kann das Augenhöhlenabdeckgitter 1 spezifisch auf den oder die die Augenhöhle ausformenden Knochen angepasst werden.

Die Oberseite des Implantats / Augenhöhlenabdeckgitters 1 ist mit dem Referenzzeichen 4 versehen. Auf dieser Oberseite 4 sind ein erster Kanal 5 sowie ein zweiter Kanal 6 ausgebildet. Beide Kanäle 5 und 6 verlaufen linear und sind optisch und taktil erkennbar. Jeder Kanal 5 bzw. 6 definiert einen Insertionsvektor. Jeder Kanal 5 bzw. 6 weist je zwei Kanalwände 7 auf, die orthogonal von der Oberseite 4 abstehen, wobei zwischen den beiden Kanalwänden 7 eines Kanals 5 bzw. 6 ein Kanalgrund 8 definiert ist.

In / an / auf dem Kanalgrund 8 ist ein Navigations-Stopp 9 vorhanden. Im ersten Kanal 5 sind zwei Navigations-Stopps 9 vorgesehen, wohingegen im zweiten Kanal 6 nur ein einziger Navigations-Stopp 9 vorgesehen ist.

Im Hauptkörper 2 sind Perforationen oder Schlitze / Durchgangsschlitze 10 nach Art von Durchgangsöffnungen vorgehalten. Sie haben eine längliche Ausprägung. Sie verlaufen jeweils orthogonal zur Abschlusskante / Einfassung 3, die durch eine Kordel 11 gebildet wird, welche einen nahezu kreisförmigen, elliptischen oder gerundeten Querschnitt aufweist. Die Kordel wirkt daher atraumatisch.

Es ist auch eine anatomische Begrenzung 12 vorhanden. Ein exakter Tränenwegsbereich 13 ist ebenfalls physisch ausgebildet und vordefiniert.

Es sind vier Durchgangslöcher 14 in einem vorderen Rand 15 des Augenhöhlenabdeckgitters 1 vorgehalten. Die Durchgangslöcher 14 definieren einen Bohrvektor, bzw. folgen einem vorbestimmten Bohrvektor. Der Bohrvektor ist operateurspezifisch. Der Bohrvektor steht schräg auf der Oberseite 4 und / oder Unterseite des Hauptkörpers 2 des Augenhöhlenabdeckgitters 1. In die Durchgangslöcher 14 sind Schrauben einbringbar, die im Knochen verankert werden können.

Am gegenüberliegenden Ende des Hauptkörpers 2 ist ein Spitzenbereich 16 vorhanden. Dort treffen sich innerhalb oder außerhalb des den Hauptkörper 2 bildenden Materials die Insertionsvektoren.

Eine durch Ziffern, wie die Zahl 15, 25 und 35 gebildete Längenskala 17 ist auf der linken Seite des ersten Kanals 5, diesem vom Spitzenbereich 16 beginnend / folgend ausgebildet. Die Längenskala 17 ist nach Art eines (kalibrierten) Lineals ausgestaltet.

Die Schlitze / Durchgangsschlitze 10 bilden ein Abflusssystem aus. Im Spitzenbereich 16 ist ein kritischer Bereich / eine critical area in puncto des Sehkanals / Sehnervs vordefiniert. Der den Kanälen 5 und 6 zugrunde liegende Insertionsvektor ist mit dem Bezugszeichen 18 versehen.

Die Kanäle 5 und 6 sind nicht nur von Vorteil beim Implantieren, also Einsetzen des Augenhöhlenabdeckgitters 1, sondern auch bei der nachfolgenden Kontrolle des Implantiervorganges. So kann ein qualitätssicherndes Vorgehen ermöglicht werden, ohne dass der Patient verletzt wird. Ein Abgleich der realen Lage des Augenhöhlenabdeckgitters 1 im Patienten mit einer gewünschten Lage am Rechner wird jederzeit einfach durchführbar. Ein postoperatives Kontrollieren der Lage wird erleichtert. Ein Übereinstimmen mit dem 3D-Datensatz, wie er geplant war, wird ermöglicht. Dazu kann der Patient mit einem Referenzpunkt versehen werden und in den Rechner eingelesen werden. Insbesondere bieten sich drei Referenzpunkte an. Die Kanäle 5 und 6 a-gieren dann als Führungsstraße mit Zwischenpunkten / Vertiefungen. Die Führungsstraße ist also der erste Kanal 5 bzw. der zweite Kanal 6 und die Zwischenpunkte / Vertiefungen sind dann die Navigations-Stopps 9. Die Kanäle 5 und 6 bilden also eine physikalische Doppelkontur /-linie zum verbesserten Führen eines Kontrollinstrumentes aus.

Der Spitzenbereich 16 kann nach Art einer umgedreht schneeschieberartigen Spitze ausgebildet sein, d.h. eine vom Sehnerv wegragende Krümmung ausbilden, so dass ein Aufspießen des Augenmuskels oder eine mechanische Irritation / Perforation des Sehnervs ausgeschlossen / vermieden wird. Perforationen, wie die Schlitze / Durchgangsschlitze 10 sind auch bewusst orthogonal zu einem patientenspezifischen Vektor, insbesondere zum Insertionsvektor 18 ausgerichtet. Der Spitzenbereich 16 ist zur Anlage mit dem Knochen vorbereitet. Der Rand, insbesondere gebildet durch die Abschlusskante / Einfassung 3 kann so geplant werden, dass das Implantat einen Überstand bildet, der mit dem Knochen in Anlage bringbar ist und / oder einen Griff für den Operateur stellt.

Es sei darauf hingewiesen, dass nach dem Fertigen des Augenhöhlenabdeckgitters 1, ein Sterilisationsschritt erfolgen kann und soll.

Während bisher sog. "Mittelwert-Implantate" geschaffen werden, also nicht patientenspezifisch ausgebildet sind, wird nun eine patientenspezifische Ausbildung möglich. Dazu kann ähnlich wie beim Aufbringen eines Leinentuchs auf einen Rechen, ein Sekundärmodell auf ein Primärmodell aufgebracht werden. Das Sekundärmodell kann ein Konglomerat aus unterschiedlichen Schichten und Formen sein. Das Separieren des Implantats vom 3D-Modell ist wünschenswert. Das Implantat kann dann ein Modell sein, etwa in Form eines standardisierten 3D-Datensatzes Form, etwa in Form eines STL-Datensatzes. Das Sekundärmodell kann ein "BMP-Template" sein, wobei auch JPEG-, TIFF- und ähnliche Formate möglich sind. Natürlich kann als Material des Augenhöhlenabdeckgitters 1 auch resorbierbares Material eingesetzt werden.

### Bezugszeichenliste

- 1: Augenhöhlenabdeckgitter
- 2: Hauptkörper
- 3: Abschlusskante / Einfassung
- 4: Oberseite
- 5: erster Kanal
- 6: zweiter Kanal
- 7: Kanalwand
- 8: Kanalgrund
- 9: Navigations-Stopp
- 10: Schlitz / Durchgangsschlitz
- 11: Kordel/ glatte Umrandung
- 12: anatomische Begrenzung
- 13: Tränenwegsbereich
- 14: Durchgangsloch
- 15: vorderer Rand
- 16: Spitzenbereich
- 17: Längenskala
- 18: Insertionsvektor

## Patentansprüche

1. Verfahren zum Herstellen eines patientenspezifisch angepassten Augenhöhlenabdeckgitters (1), wobei ein 3D-Primärmodell der abzudeckenden oder ersetzenden Knochenstruktur im Bereich einer Augenhöhle eines zu behandelnden Patienten geschaffen wird, danach ein für die maximale räumliche Ausdehnung des geplanten Augenhöhlenabdeckgitters (1) repräsentativer Grenzbereich festgelegt wird, danach ein 2D-Sekundärmodell innerhalb des Grenzbereichs so auf das 3D-Primärmodell überführt wird, dass die geometrische Beschaffenheit des 3D-Primärmodells auf die Ausgangsform des ursprünglichen 2D-Sekundärmodells übertragen zu einem 3D-Tertiärmodell führt, auf dessen Basis die Fertigung des Augenhöhlenabdeckgitters (1) basiert.

2. Verfahren nach Anspruch 1, **dadurch gekennzeichnet, dass** bei der Fertigung generative Verfahren, wie Sinterverfahren, und/oder CNC-, Fräs- oder Spitzgussverfahren angewandt werden.

3. Verfahren nach einem der Ansprüche 1 oder 2, **dadurch gekennzeichnet, dass** das Augenhöhlengitter (1) nur aus einem oder mehreren Metallwerkstoff(en) oder nur aus Kunststoff oder einem Gemisch aus Metall und Kunststoff besteht.

4. Verfahren nach einem der Ansprüche 1 bis 3, **dadurch gekennzeichnet, dass** das 2D-Sekundärmodell aus mehreren Schichten aufgebaut / zusammengesetzt wird / ist.

## Claims

1. Method of producing a patient-specific eye cavity cover grid (1), wherein a 3D primary model is formed of the bone structure to be covered or replaced in the region of an eye cavity of a patient to be treated, then a border region representative of the maximum spatial extension of the planned eye cavity cover grid (1) is established, then a 2D secondary model is positioned within the border region over the 3D primary model so that the geometric dimensions of the 3D primary model transferred to the starting form of the original 2D secondary model lead to a 3D tertiary model which is used as the basis for the production of the eye cavity cover grid (1).

2. Method according to claim 1, **characterized in that** generative methods, such as sintering methods, and/or CNC, milling, or injection molding methods are applied during production.

3. Method according to one of claims 1 or 2, **characterized in that** the eye cavity grid (1) is made solely of one or more metallic materials, or solely of plastic, or a mixture of metal and plastic.

4. Method according to one of claims 1 to 3, **characterized in that** the 2D secondary model is constructed/composed of a plurality of layers.

## Revendications

1. Procédé de fabrication d'une grille de recouvrement d'orbite (1) adaptée de manière spécifique au patient, un modèle primaire 3D de la structure osseuse à recouvrir ou à remplacer étant créé dans la zone d'une orbite d'un patient à traiter, une zone limite représentative de l'extension spatiale maximale de la grille de recouvrement d'orbite (1) prévue étant ensuite déterminée, un modèle secondaire 2D étant ensuite transféré au modèle primaire 3D à l'intérieur de la zone limite de sorte que la nature géométrique du modèle primaire 3D transmis à la forme de départ du modèle secondaire 2D d'origine mène à un modèle tertiaire 3D, sur la base duquel la fabrication de la grille de recouvrement d'orbite (1) se fonde.

2. Procédé selon la revendication 1, **caractérisé en ce que** lors de la fabrication, des procédés génératifs, tels que des procédés de frittage et/ou procédés CNC, de fraisage ou de moulage par injection sont appliqués.

3. Procédé selon l'une quelconque des revendications 1 ou 2, **caractérisé en ce que** la grille d'orbite (1) se compose seulement d'un ou de plusieurs matériaux métalliques ou seulement d'un plastique ou d'un mélange de métal et de plastique.

4. Procédé selon l'une quelconque des revendications 1 à 3, **caractérisé en ce que** le modèle secondaire 2D est structuré à partir de/composé de plusieurs couches.
